# EUROPEAN PATENT APPLICATION

(11) **EP 2 749 287 A1**
(43) Date of publication of application: **02.07.2014**
(21) Application number: 11871336.1
(22) Date of filing: 24.08.2011
(51) Int. Cl.: A61K 36/18, A23L 1/30, A61P 31/16

(54) **INFLUENZA VIRUS INFECTION INHIBITOR**

(71) Applicant: Lotte Co., Ltd., Tokyo 160-0023 (JP)
(72) Inventor: ISHIZUKA, Tomokazu, Saitama-shi Saitama 336-8601 (JP); KIYONO, Hiroshi, Tokyo 113-0033 (JP); KUNISAWA, Jun, Tokyo 113-0033 (JP); YOSHIDA, Keishiro, Saitama-shi Saitama 336-8601 (JP); SHIMURA, Susumu, Saitama-shi Saitama 336-8601 (JP)
(74) Representative: Blodig, Wolfgang
(86) International application number: PCT/JP2011/004701
(87) International publication number: WO 2013/027245

(57) **Abstract**

A composition having infection inhibitory effects against influenza viruses is provided. An antiinfluenza virus agent comprises as an active ingredient an extract obtained by extracting a corn poppy with water, and then freeze-drying the extract for purification.

## Description

### Technical Field

Provision of a composition which has infection inhibitory effects against influenza viruses.

### Background Art

A person infected with an influenza virus shows clinical symptoms such as high fever, headache, arthralgia, general malaise, or the like, and, dies in some cases. The influenza viruses are highly infectious, and characterized in causing the pandemic in a short period of time unlike the common cold. Prevention by the vaccination and the treatment by drugs such as neuraminidase inhibitor, or the like, are commonly known as the methods for preventing and treating influenza. However, vaccines are not versatile since the effect thereof significantly varies depending on the type of influenza virus, whereas drugs pose problems of adverse effects and resistant virus emergence.

Under such a circumstance, compositions for preventing or treating the infection of influenza viruses have been developed, and Kakkon-to (NPL 1), lactoferrin and lactoperoxidase (NPL 2), Bacillus coagulans (PTL 1), cromoglycic acid (PTL 2), colostrum-derived protein fraction of mammals (PTL 3), sialoglyco conjugates-containing polymer (PTL 4), buckwheat (PTL 5), cassis (PTL 6), various plant extracts (PTLs 7 and 8), and the like, are disclosed as the components exhibiting the influenza virus infection inhibitory effect. However, these components are not assured to demonstrate satisfactory influenza virus infection inhibitory effects so far, and hence further prevention and treatment methods are expected to be developed.

The present inventors conducted searches for the purpose of providing a substance which exhibits influenza virus infection inhibitory effects and found that a corn poppy extract demonstrates a valid influenza virus infection inhibitory effect. corn poppy (scientific name: Papaver rhoeas L.), identified by the present invention to have the infection inhibitory effect against influenza viruses, is a plant belonging to the Papaveraceae Family, also known as Gubijinso, which is believed to have sedative effect, hypnotic effect, analgesic effect, and the like, and hence used as an antitussive and sedative but is not known of the virus infection inhibitory effect.

The pharmacological effect of poppy is described in the patent pertinent to immunostimulants including herbs (PTL 9) as an example of the herbs, but the literature lacks in the concreteness of the invention and does not describe or suggest virus infection inhibitory effects of poppy.

### Citation List

### Patent Literature

PTL 1: Japanese Patent Application Laid-Open No. 2008-13543
PTL 2: Japanese Patent Application Laid-Open No. 2004-352712
PTL 3: Japanese Patent Application Laid-Open No. 2009-190994
PTL 4: Japanese Patent Application Laid-Open No. 2008-31156
PTL 5: Japanese Patent No. 4185996
PTL 6: Japanese Patent Application Laid-Open No. 2009-269861
PTL 7: Japanese Patent Application Laid-Open No. 2005-343836
PTL 8: Japanese Patent Application Laid-Open No. 2004-59463
PTL 9: Japanese Patent Application Laid-Open No. 2002-370993

### Non Patent Literature

NPL 1: Kurokawa M. et al., Antiviral Research, 2002, Vol. 56, pages 183-188, Effect of interleukin-12 level augmented by Kakkon-to, a herbal medicine, on the early stage of influenza infection in mice.
NPL 2: Shin K. et al. Journal of Medical Microbiology, 2005, Vol. 54, pages 717-723, Effects of orally administered bovine lactoferrin and lactoperoxidase on influenza virus infection in mice.

### Summary of Invention

### Technical Problem

The present invention provides a composition having infection inhibitory effects against influenza viruses.

### Solution to Problem

The present invention relates to an antiinfluenza virus agent comprising a corn poppy extract as an active ingredient.

Further, the present invention relates to an antiinfluenza virus agent comprising as an active ingredient an extract obtained by extracting a corn poppy with water, and then freeze-drying the extract for purification.

Furthermore, the present invention relates to a drink or food product comprising a corn poppy extract having an antiinfluenza virus effect.

### Advantageous Effects of Invention

The corn poppy extract of the present invention has an IgA secretion promoting effect and influenza virus infection inhibitory effect and is thus considered to make a body resistant to influenza infection, and is a new material capable of reinforcing the function of candies.

### Brief Description of Drawings

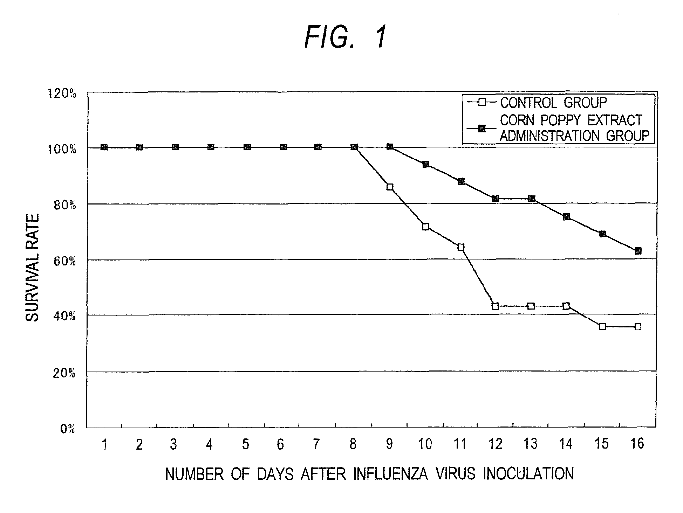

FIG. 1 is a graph showing the mouse survival rates of a corn poppy extract administration group and a control group up to Day 16 after influenza virus inoculation.

### Description of Embodiments

The present inventors, as a result of searches for a substance which exhibits influenza virus infection inhibitory effects, investigated a corn poppy extract and found that, when the mice kept with and without administration of the corn poppy extract were infected with the same dose of an influenza virus and monitored for the development over time, the mice administered with the corn poppy extract showed a milder body weight loss caused by the influenza virus infection and had a tendency of improved survival rate.

Hereinbelow, specific examples are described with reference to Examples. However, the present invention is not limited to the specific examples below.

### Example 1

### Preparation of a corn poppy hot-water extract

After pulverizing 100 g of corn poppy (flower), water in an amount of 10 times the weight of the sample was added to extract at 70°C for 2 hours. After filtering insoluble matters, the obtained extract was freeze-dried, thereby obtaining 37.5 g of a corn poppy hot-water extract.

### Example 2

Influenza virus infection inhibitory effect by repeated sublingual administration to mouse 7 Week-old 30 mice (BALB/c) were divided into a control group (14 mice) and an administration group (16 mice), and kept on a common diet (MF, Oriental Yeast). The corn poppy (flower) hot-water extract was sublingually administered in the form of aqueous solution to the administration group every day from the start of the feeding in a dose of 4 mg/day, whereas the same amount of only water was sublingually administered to the control group. On week 4, an influenza virus was transnasally inoculated under anesthesia (100 pfu/50 µl PBS/mouse) and the body weights up to Day 16 after inoculation were measured. An influenza virus A/PR/8/34 (H1N1) strain was used, and the corn poppy (flower) hot-water extract was also sublingually administered after infecting the virus. The body weight of each mouse at the time of inoculating the virus is as shown in Table 1 below, with no significant difference found between both groups.

[Table 1]

**Table 1. Body weight of each animal at the time of virus inoculation**

| Control group | | Corn poppy extract administration group | |
|---|---|---|---|
| Animal | Body weight (g) | Animal | Body weight (g) |
| C-1 | 20.1 | P-1 | 20.8 |
| C-2 | 20.1 | P-2 | 21.6 |
| C-3 | 22.2 | P-3 | 21.6 |
| C-4 | 20.8 | P-4 | 20.7 |
| C-5 | 21.9 | P-5 | 21.4 |
| C-6 | 21.7 | P-6 | 21.6 |
| C-7 | 21.0 | P-7 | 21.8 |
| C-8 | 22.1 | P-8 | 22.9 |
| C-9 | 23.5 | P-9 | 21.9 |
| C-10 | 21.4 | P-10 | 21.8 |
| C-11 | 21.1 | P-11 | 22.0 |
| C-12 | 22.8 | P-12 | 22.9 |
| C-13 | 21.1 | P-13 | 21.7 |
| C-14 | 20.9 | P-14 | 22.0 |
| | | P-15 | 21.1 |
| | | P-16 | 23.4 |
| Average value ± standard deviation | 21.5±0.93 | Average value ± standard deviation | 21.8±0.71 |

The results obtained in the present Examples are shown in Table 2, Table 3 and FIG. 1.
Table 2 and Table 3 show the body weight changes of the mice in grams up to Day 16 after the influenza virus inoculation, and present the individual data and average data of the corn poppy extract administration group and the control group.
FIG. 1 shows the plots of mouse survival rates of the corn poppy extract administration group and the control group up to Day 16 after the influenza virus inoculation.
In Table 2 and Table 3, the number of days indicates the number of days from the influenza virus inoculation, and the body weight change indicates the amount changed from the body weight at the time of the virus inoculation. The x in Table 2 and Table 3 indicates that a mouse died.

As evident in FIG. 1, on Day 16 after the influenza virus inoculation, the control group had 5 mice survived out of 14 mice (survival rate 36%), whereas the corn poppy extract administration group had 10 mice survived out of 16 mice (survival rate 63%). The results of log-rank test showed that the administration group had a significantly higher survival rate than the control group up to Day 12, and the administration group had a tendency of higher survival rate than the control group up to Day 16.
Further, for the body weight change, as a result of t-test, the administration group had a more significantly inhibited body weight loss than the control group during the period from Day 3 to Day 7, and thus the administration group was considered to have a milder body weight loss caused by the virus infection.

It is conceived from the above results that the death and body weight loss of the mice caused by the virus infection are inhibited by the administration of corn poppy extract.

[Table 2]

**Table 2**

| Group | Animal | Body weight change (g) | | | | | | | | | | | | | |
|---|---|---|---|---|---|---|---|---|---|---|---|---|---|---|---|
| | | Day 1 | Day 2 | Day 3 | Day 4 | Day 6 | Day 7 | Day 8 | Day 9 | Day 10 | Day 11 | Day 12 | Day 14 | Day 15 | Day 16 |
| Control group | C-1 | 0.40 | -0.20 | -1.60 | -2.60 | -4.00 | -4.90 | -5.90 | -7.00 | -7.30 | -7.60 | × | | | |
| | C-2 | 0.70 | 0.50 | -1.10 | -2.20 | -4.30 | -5.20 | -6.10 | -7.10 | -7.30 | × | | | | |
| | C-3 | 0.30 | 0.20 | -2.00 | -2.90 | -4.90 | -6.00 | -6.90 | -7.00 | -7.40 | -7.80 | × | | | |
| | C-4 | 0.50 | 0.30 | -1.30 | -2.30 | -4.30 | -5.40 | -5.90 | -6.20 | -6.20 | -6.40 | -6.30 | -5.70 | -5.20 | -4.90 |
| | C-5 | -0.10 | -0.30 | -2.20 | -3.10 | -5.00 | -6.00 | -6.80 | -6.70 | -6.50 | -6.30 | -6.40 | -5.80 | -5.40 | -4.40 |
| | C-6 | 0.20 | -0.30 | -2.00 | -2.60 | -4.60 | -5.70 | -6.70 | × | | | | | | |
| | C-7 | 0.90 | 0.20 | -2.10 | -3.40 | -5.30 | -6.00 | -6.00 | -6.50 | -6.70 | -7.30 | -7.50 | -7.60 | -7.70 | -7.60 |
| | C-8 | 0.40 | 0.30 | -1.90 | -3.90 | -6.20 | -7.00 | -7.40 | × | | | | | | |
| | C-9 | 1.40 | 0.90 | -1.20 | -2.70 | -5.10 | -6.10 | -7.00 | -7.90 | × | | | | | |
| | C-10 | -0.70 | -0.30 | -2.40 | -3.90 | -5.80 | -6.40 | -7.00 | -6.70 | -6.50 | -6.20 | -5.20 | -3.50 | -3.20 | -2.50 |
| | C-11 | 0.40 | 0.20 | -2.30 | -3.50 | -5.60 | -6.00 | -6.40 | -7.10 | × | | | | | |
| | C-12 | 0.00 | 0.20 | -1.40 | -2.90 | -5.90 | -6.80 | -6.90 | -7.00 | -7.30 | -7.10 | -6.80 | -5.60 | -4.90 | -4.10 |
| | C-13 | 0.30 | 0.20 | -1.40 | -2.80 | -4.40 | -5.40 | -5.80 | -6.50 | -6.80 | -7.10 | -7.70 | -7.90 | × | |
| | C-14 | 0.60 | 0.30 | -1.00 | -2.30 | -4.30 | -5.40 | -6.00 | -6.40 | -6.40 | -6.10 | × | | | |
| | Average | 0.38 | 0.16 | -1.71 | -2.94 | -4.98 | -5.88 | -6.49 | -6.84 | -6.84 | -6.88 | -6.65 | -6.02 | -5.28 | -4.70 |

[Table 3]

**Table 3**

| Group | Animal | Body weight change (g) | | | | | | | | | | | | | |
|---|---|---|---|---|---|---|---|---|---|---|---|---|---|---|---|
| | | Day 1 | Day 2 | Day 3 | Day 4 | Day 6 | Day 7 | Day 8 | Day 9 | Day 10 | Day 11 | Day 12 | Day 14 | Day 15 | Day 16 |
| Corn poppy extract administration group | P-1 | 0.30 | 0.50 | -0.30' | -1.90 | -3.80 | -4.90 | -5.50 | -5.50 | -5.60 | -5.90 | -6.00 | -6.30 | -6.00 | -5.60 |
| | P-2 | 0.40 | 0.20 | -0.30 | -0.90 | -2.80 | -4.10 | -5.10 | -5.00 | -4.90 | -4.70 | -4.70 | -2.30 | -1.20 | -0.80 |
| | P-3 | 0.10 | -0.10 | -1.60 | -2.40 | -4.20 | -5.40 | -5.90 | -6.00 | -5.80 | -5.20 | -4.40 | -2.40 | -1.90 | -1.90 |
| | P-4 | 0.30 | 0.10 | -0.10 | -0.40 | -1.40 | -2.60 | -4.00 | -4.70 | -4.60 | -4.10 | -3.40 | -1.20 | -0.60 | -0.50 |
| | P-5 | 0.60 | 0.00 | -1.70 | -2.40 | -4.10 | -5.20 | -6.10 | -6.50 | -6.60 | -6.70 | -7.00 | -7.10 | -7.10 | -7.00 |
| | P-6 | 0.30 | -0.30 | -1.50 | -2.40 | -4.60 | -5.70 | -6.70 | -7.10 | -7.30 | -7.70 | -8.10 | -9.20 | × | |
| | P-7 | 0.40 | 0.10 | -0.80 | -1.70 | -3.10 | -4.50 | -5.80 | -5.90 | -5.70 | -5.70 | -5.80 | -4.80 | -3.70 | -2.90 |
| | P-8 | -0.20 | -0.50 | -1.40 | -2.40 | -4.50 | -5.40 | -6.70 | -7.50 | -7.40 | -7.50 | -7.90 | -8.60 | -9.00 | -9.00 |
| | P-9 | 0.10 | 0.60 | -1.80 | -3.10 | -5.60 | -6.00 | -7.00 | -7.50 | × | | | | | |
| | P-10 | 0.50 | 0.40 | -0.90 | -2.10 | -4.60 | -5.60 | -6.30 | -6.80 | -7.20 | -7.50 | -7.80 | × | | |
| | P-11 | 0.40 | 0.00 | -1.20 | -2.10 | -4.30 | -5.60 | -6.50 | -7.10 | -7.20 | -7.30 | -7.80 | -8.10 | -8.40 | × |
| | P-12 | 0.70 | 0.60 | -1.10 | -2.50 | -4.80 | -5.80 | -6.70 | -7.80 | -8.40 | -8.80 | × | | | |
| | P-13 | 0.70 | 0.50 | -0.80 | -2.60 | -4.70 | -5.70 | -6.60 | -6.90 | -7.50 | -7.30 | -7.20 | -7.40 | -7.70 | -7.50 |
| | P-14 | 0.80 | 1.00 | 0.00 | -2.10 | -4.40 | -5.40 | -6.40 | -7.00 | -7.30 | × | | | | |
| | P-15 | 1.30 | 1.20 | 0.20 | -1.60 | -4.50 | -5.30 | -6.10 | -7.10 | -7.20 | -7.50 | -7.70 | -7.90 | -8.10 | -8.10 |
| | P-16 | -0.30 | 0.60 | -0.30 | -2.70 | -5.50 | -6.40 | -7.50 | -7.70 | -7.80 | -7.40 | -7.50 | -7.80 | -8.10 | -8.00 |
| | Average | 0.40 | 0.31 | -0.85 | -2.08 | -4.18 | -5.23 | -6.18 | -6.63 | -6.70 | -6.66 | -6.56 | -6.09 | -5.62 | -5.13 |

Next, using the corn poppy extract of the present invention which demonstrated the influenza virus infection inhibitory effect, a tablet, chewing gum, candy, chocolate, biscuit, gummy jelly, tablet candy, ice cream, sorbet, and drink were prepared by a routine method. The formulae are shown below.

### Example 3

A tablet was prepared in accordance with the following formula.

| | |
|---|---|
| D-mannitol | 33.6% |
| Lactose | 33.6 |
| Crystalline cellulose | 8.5 |
| Hydroxypropylcellulose | 4.3 |
| Corn poppy extract | 20.0 |
| | 100.0% |

### Example 4

A chewing gum was prepared in accordance with the following formula.

| | |
|---|---|
| Gum base | 20.0% |
| Sugar | 54.7 |
| Glucose | 15.0 |
| Starch syrup | 9.5 |
| Flavor | 0.5 |
| Corn poppy extract | 0.3 |
| | 100.0% |

### Example 5

A candy was prepared in accordance with the following formula.

| | |
|---|---|
| Sugar | 50.0% |
| Starch syrup | 33.0 |
| Citric acid | 1.0 |
| Flavor | 0.2 |
| L-Menthol | 1.0 |
| Corn poppy extract | 0.5 |
| Water | 14.3 |
| | 100.0% |

### Example 6

A chocolate was prepared in accordance with the following formula.

| | |
|---|---|
| Cacao bitter | 20.0% |
| Whole milk powder | 20.0 |
| Cacao butter | 17.0 |
| Powdered sugar | 41.85 |
| Lecithin | 0.45 |
| Flavor | 0.1 |
| Corn poppy extract | 0.6 |
| | 100.0% |

### Example 7

A biscuit was prepared in accordance with the following formula.

| | |
|---|---|
| Sugar | 31.7% |
| Flour | 26.8 |
| Potato starch | 26.8 |
| Butter | 3.2 |
| Egg | 10.2 |
| Sodium bicarbonate | 0.3 |
| Corn poppy extract | 1.0 |
| | 100.0% |

### Example 8

A gummy jelly was prepared in accordance with the following formula.

| | |
|---|---|
| Polydextrose aqueous solution | 40.0% |
| Sorbitol aqueous solution | 8.0 |
| Palatinose aqueous solution | 9.0 |
| Maltose aqueous solution | 20.0 |
| Trehalose aqueous solution | 11.0 |
| Gelatin | 10.0 |
| Tartaric acid | 1.0 |
| Corn poppy extract | 1.0 |
| | 100.0% |

### Example 9

A tablet candy was prepared in accordance with the following formula.

| | |
|---|---|
| Sugar | 76.1% |
| Glucose | 19.0 |
| Sucrose esters of fatty acids | 0.2 |
| Flavor | 0.2 |
| Corn poppy extract | 0.5 |
| Water | 4.0 |
| | 100.0% |

### Example 10

A tablet was prepared in accordance with the following formula.

| | |
|---|---|
| Sugar | 35.85% |
| Sucrose esters of fatty acids | 0.15 |
| Corn poppy extract | 60.0 |
| Water | 4.0 |
| | 100.0% |

### Example 11

An ice cream was prepared in accordance with the following formula.

| | |
|---|---|
| Egg yolk | 11.0% |
| Sugar | 14.0 |
| Cow's milk | 37.0 |
| Fresh cream | 37.0 |
| Vanilla bean | 0.5 |
| Corn poppy extract | 0.5 |
| | 100.0% |

### Example 12

A sorbet was prepared in accordance with the following formula.

| | |
|---|---|
| Orange fruit juice | 16.0% |
| Sugar | 31.0 |
| Corn poppy extract | 3.0 |
| Water | 50.0 |
| | 100.0% |

### Example 13

A drink was prepared in accordance with the following formula.

| | |
|---|---|
| Orange fruit juice | 30.0% |
| Isomerized syrup | 15.33 |
| Citric acid | 0.1 |
| Vitamin C | 0.04 |
| Flavor | 0.1 |
| Corn poppy extract | 0.01 |
| Water | 54.42 |
| | 100.0% |

### Industrial Applicability

The corn poppy extract of the present invention demonstrates the influenza virus infection inhibitory effect and is hence applicable to various pharmaceutical products and drink and food products for inhibiting influenza viruses.

## Claims

1. An antiinfluenza virus agent comprising a corn poppy extract as an active ingredient.

2. An antiinfluenza virus agent comprising as an active ingredient an extract obtained by extracting a corn poppy with water, and then freeze-drying the extract for purification.

3. A drink or food product comprising a corn poppy extract having an antiinfluenza virus effect.
